# EUROPÄISCHE PATENTSCHRIFT

(11) **EP 4 406 509 B1**
(45) Veröffentlichungstag und Bekanntmachung des Hinweises auf die Patenterteilung: **14.01.2026**
(21) Anmeldenummer: 23153848.9
(22) Anmeldetag: 30.01.2023
(51) Int. Cl.: A61C 17/02, A61L 2/03, A61L 2/00, A61L 2/025, A61C 17/20, A61C 17/024

(54) **REINIGUNGSVORRICHTUNG ZUR ENTFERNUNG VON BIOFILMEN IM DENTALBEREICH**
CLEANING DEVICE FOR REMOVING BIOFILMS IN THE DENTAL FIELD
DISPOSITIF DE NETTOYAGE POUR ÉLIMINER DES BIOFILMS DANS LE DOMAINE DENTAIRE

(43) Veröffentlichungstag der Anmeldung: 31.07.2024
(73) Patentinhaber: Justus-Liebig-Universität Gießen, Körperschaft des öffentlichen Rechts, 35390 Gießen (DE)
(72) Erfinder: Meyle, Jörg, 35444 Biebertal (DE); Helbig, Bernd, 34621 Fielendorf (DE)
(74) Vertreter: Stumpf, Peter

(56) Entgegenhaltungen:
- EP-A1- 3 323 380
- WO-A1-2021/018871
- JP-A- 2013 141 601

## Beschreibung

Die vorliegende Erfindung betrifft eine Reinigungsvorrichtung zur Entfernung von Biofilmen im Dentalbereich insbesondere auf Zähnen und Zahnimplantaten.

### Beschreibung und Einleitung des allgemeinen Gebietes der Erfindung

Die Reinigung und Dekontamination entzündeter Zahnfleischtaschen ist das oberste Ziel der Parodontaltherapie, wobei mithilfe von Hand-, Schall- bzw. Ultraschallinstrumenten, Pulver-/Wasserstrahlgeräten oder Lasern versucht wird, den bakteriellen Biofilm aus den entzündeten Taschen zu entfernen.

### Stand der Technik

Für die Entfernung des bakteriellen Biofilms gibt es seit vielen Jahren außer Handinstrumenten Schall- bzw. Ultraschallgeräte, mit denen der Biofilm und der Zahnstein von den Zahn-, Wurzel bzw. Implantatoberfläche abgelöst werden kann. Es stehen dafür auch Pulver-Wasserstrahl Geräte bzw. Laser zur Verfügung.

Mit Pulver-Wasserstrahl-Geräten (u.a. Fa. Elektro- Medical Systems EMS, Nyon, Schweiz), wird ein feinkörniges Pulver zusammen mit einem Luft- Wassergemisch auf die Oberfläche aufgesprüht.

Es existieren auch bereits elektrochemische Lösungen zum Reinigen von frei zugänglichen Dentalimplantaten. Dabei wird der Biofilm bzw. werden die Keime ausgehend von der Implantatoberfläche getötet und/oder entfernt, indem in einem als Behandlungsflüssigkeit dienenden Fluid gelöste Ionen (Kationen und/oder Anionen) mittels elektrostatischer Kräfte durch den Biofilm hindurch zur Implantatoberfläche befördert werden, um dort den Biofilm zu entfernen und so das Implantat zu reinigen.

Die keimtötende Wirkung dieses Prozesses basiert auf unterschiedlichen Effekten. Zum einen werden durch das Anlegen einer elektrischen Spannung Ionen aus dem Biofilm selbst (auch aus den Bakterien) zur Anode oder Kathode befördert. Dies kann zur Abtötung von Bakterien führen. Darüber hinaus können die Ionen, während sie den Biofilm passieren, biochemische Reaktionen eingehen, was ebenfalls zur Abtötung von Bakterien führen kann. In Folge der elektrischen Spannung bilden sich auf der Implantatoberfläche durch Elektrolyse des Wassers kleine Gasbläschen die von der Materialoberfläche ausgehen, wodurch die auf der Oberfläche angehefteten Bakterien abgelöst werden. Eine weitere Möglichkeit der Abtötung besteht darin, dass die an der Implantatoberfläche neu gebildeten Stoffverbindungen antibakterielle und/oder antivirale und/oder fungizide Wirkung besitzen. Dies kann natürlich auch passieren, wenn die Ionen in den atomaren Zustand übergehen.

In der Schrift EP3323380A1 wird ein Behandlungssystem zur Reinigung eines mit Biofilm verunreinigten Bauteils, insbesondere zur Reinigung von bakteriell verunreinigten Oberflächen von Knochenimplantaten oder Dentalimplantaten vorgeschlagen.

In der Schrift JP2013141601A wird ein ionengesteuertes Zahnfleischspalt-Reinigungssystem beschrieben. Dieses umfasst: eine Zahnfleischsulkus-Reinigungsvorrichtung, die einen Ioneninduktions-Leitungsschaltkreis enthält beschrieben. Dabei wird eine wässrige Lösung einer wasserlöslichen Substanz verwendet, die eine elektrische Polarität und eine anitimikrobielle Wirkung aufweist. Bei der Reinigung der Innenseite eines Zahnfleischsulkus wird durch Ablassen des Reinigungswassers mit niedrigem Druck aus einer in einem Handstück installierten Düse ein loneninduktionskreis, der die Innenseite eines lebenden Organismus und einen Reinigungswasserablasspfad einschließt, geschlossen. Dabei entsteht ein Ioneninduktions-Energiestromkreis, der durch Verbinden einer Elektrode auf der Seite des lebenden Organismus und einer Elektrode auf der Seite des Reinigungswassers mit einer Batterie konfiguriert ist. Dabei wird eine bakteriostatische Ionensubstanz elektrisch induziert und auf einem Gewebe um die Innenseite des Zahnfleischsulkus adsorbiert und als Mittel zur Unterdrückung der Existenz und Vermehrung von Mikroorganismen im Zahnfleischsulkus verwendet.

Die Schrift WO2021018871A1 beschreibt ein Kontaktierungssystem zur elektrischen Kontaktierung von in einen isolierenden Mantel eines Kabelstrangs eingebetteten elektrischen Leiterelementen. Es soll auf besonders einfache und kostengünstige Weise eine auch hohen Zuverlässigkeitsanforderungen genügende elektrische Kontaktierung des Kabelstrangs ermöglichen. Dies wird mit einem Anschlussmodul, in dessen Außengehäuse realisiert, in dem ein Teilabschnitt des Kabelstrangs fixierbar ist. Weiterhin wird dies mit einer Anzahl von Kontaktstiften, die von der Seite her relativ zur Längsrichtung des Kabelstrangs quer durch das Außengehäuse hindurchgeführt sind, erreicht.Dies gelingt nicht in allen Fällen, da die Geometrie und Topographie der entzündeten Bereiche sehr irregulär sein können, sodass in Nischen noch Reste von Biofilm zurückbleiben, die dann zu einer Reinfektion und einem Erkrankungsrezidiv führen können. Weiterhin sind bisherige elektrochemisch arbeitende Reinigungsvorrichtungen nur für das Reinigen von elektrisch leitfähigen Implantaten geeignet.

### Aufgabe

Aufgabe der vorliegenden Erfindung ist es Biofilme im Dentalbereich insbesondere von der Zahn- bzw. Wurzeloberfläche oder Implantatoberflächen abzulösen und zusätzlich durch chemische Interaktionen den Biofilm zu beseitigen.

### Lösung der Aufgabe

Diese Aufgabe wird erfindungsgemäß gelöst durch die erfindungsgemäße Reinigungsvorrichtung zur Entfernung von Biofilmen auf Zähnen oder Dentalimplantaten gemäß Anspruch 1.

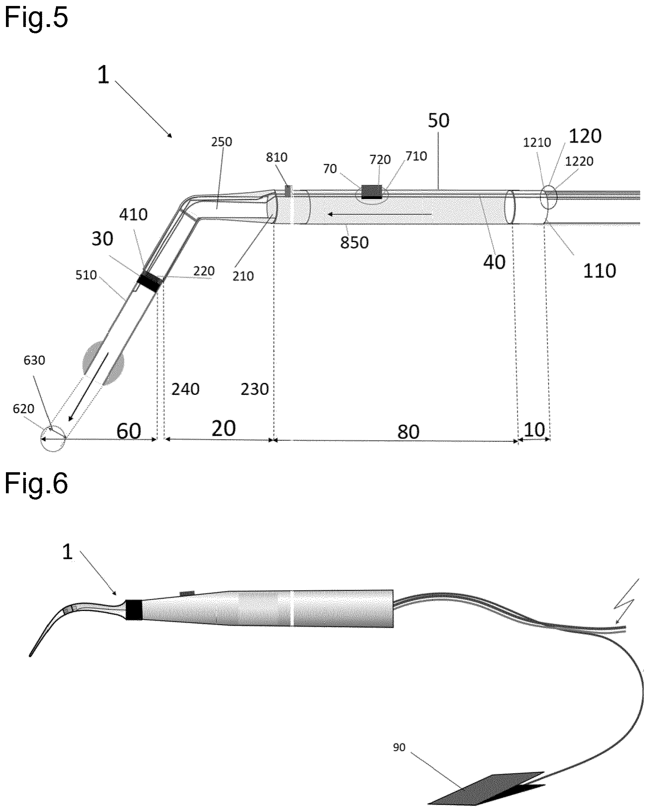

Wenn im Folgenden von Zahn gesprochen wird, meint dies einen natürlichen Zahn oder einen künstlichen Zahn bzw. ein Zahnimplantat im Mundraum. Zu reinigender Dentalbereich 2 meint im Folgenden den Abschnitt der Zähne, welcher von Biofilmen befreit werden soll. Dies ist auch in der Abbildung Fig 5 gezeigt.

Die erfindungsgemäße Reinigungsvorrichtung 1 zur Entfernung von Biofilmen im Dentalbereich umfasst wenigstens die folgenden Bauelemente

### Anschluss 10

Die Reinigungsvorrichtung 1 umfasst dabei einen Anschluss 10 um die Reinigungsvorrichtung 1 mit Fluid und elektrischer Energie zu versorgen. Der Anschluss 10 umfasst dazu wenigstens einen ersten Adapter 110 um die Reinigungsvorrichtung 1 mit Fluid (z.B. Wasser) aus einer Fluidquelle zu versorgen und wenigstens einen zweiten Adapter 120 mit einem ersten elektrischen Pol 1210 und einem zweiten elektrischen Pol 1220, um die Reinigungsvorrichtung 1 mit elektrischer Energie aus einer Stromquelle zu versorgen. Im Sinne der klinischen Praktikabilität wird dabei ein möglichst einfaches Anschlusssystem z.B. Klicktechnik o.ä. präferiert, sodass der Rest der Reinigungsvorrichtung auch als Einmalprodukt verwendet werden kann. Alternativ kann der Anschluss auch als Bajonettverschluss ausgeführt sein. Der erste elektrische Pol 1210 und der zweite elektrische Pol 1220 können dabei jeweils als Anode oder Kathode fungieren.

### Schaft 20

Weiterhin umfasst die Reinigungsvorrichtung 1 einen fluiddurchströmbaren Schaft 20. Der fluiddurchströmbare Schaft 20 umfasst wenigstens eine erste Öffnung 210 an seinem ersten Ende 230 zum Verbinden des Schafts 20 mit dem Adapter 110 des Anschluss 10.Weiterhin umfasst der Schaft 20 ein zweites Ende 240. Dabei ist das zweite Ende 240 des Schafts dem ersten Ende 230 des Schaftes 20 entgegengesetzt angeordnet.

Weiterhin umfasst der Schaft wenigstens eine zweite Öffnung 220 in Richtung des zu reinigenden Dentalbereich 3. Der fluiddurchströmbare Schaft 20 kann auch mehrere Öffnungen 220 aufweisen. Größe und Anzahl der zweiten Öffnungen 220 sind abhängig von der Fließgeschwindigkeit und der Fluidaustrittsmenge der Grundeinheit. Typische Flussraten liegen im Bereich von ca. 50 - 130 ml je min. Die Anzahl der Öffnungen 220 liegt vorzugsweise bei 1 bis 10 besonders bevorzugt 2 bis 4 und ihr Durchmesser zwischen 0,1 mm und 2 mm.

Zwischen der wenigstens einen ersten Öffnung 210 und der wenigstens einen zweiten Öffnung 220 befindet sich ein Hohlraum 250. Durch diesen Hohlraum 250 kann ein Fluid frei strömen.

Der fluiddurchströmbare Schaft 20 ist dabei mit dem Anschluss 10 so verbindbar, dass ein Fluid durch den Adapter 110 über die erste Öffnung 210 und den Hohlraum 250 durch wenigstens eine zweite Öffnung 220 strömen kann.

In einer besonderen Ausführungsform ist die wenigstens eine Öffnung 220 längsoval ausgebildet, sodass die Flüssigkeitsrichtung zur Spitze 60 orientiert ist.

Die Größe des Schafts 20 richtet sich nach dem dentalen Einsatzfeld und liegt üblicherweise im Bereich 1 bis 5 cm. Als Material wird ein üblicher OP-geeigneter Kunststoff beispielsweise PEEK (Polyetheretherketon) oder Edelstahl bevorzugt.

Das zweite Ende 240 des Schafts besteht dabei aus vorzugsweise aus einem nichtleitenden Material z.B. Kunststoff oder (Zirkon-)Keramik. Als Kunststoff kommen u.a. Duroplaste in Frage, die nicht leitfähig sind und sterilisiert zumindest thermodesinfiziert werden können. Sterilisierung ist in DIN EN 285 näher definiert. Gemäß DIN EN 285 müssen alle Oberflächen der sterilisierten Gegenstände einem reinen, gesättigten Wasserdampf mit einer Temperatur von 134 °C über mindestens drei Minuten ausgesetzt werden. Thermodesinfektion ist ein in der Zahnmedizin gängiges Verfahren zur Reinigung von Instrumenten, das für verschiedene Instrumente angewendet wird.

### Isolator 30

Weiterhin umfasst die Reinigungsvorrichtung 100 einen Isolator 30. Dieser ist am zweiten Ende 240 des Schaftes angeordnet. Dabei ist das zweite Ende 240 des Schafts dem ersten Ende 230 des Schaftes 20 entgegengesetzt angeordnet.

Dabei ist der Isolator 30 mit dem Schaft 20 verbindbar ausgebildet. Die Verbindung kann dabei beispielsweise durch kleben oder Presssitz erfolgen. Alternativ kann die Verbindung über einen Konus 330 des Isolators erfolgen.

Dabei weist der Isolator 30 eine Bohrung 310 auf, durch welche eine elektrische Leitung durch den Isolator 30 und Fluid geführt werden kann. Der Durchmesser der Bohrung beträgt vorzugsweise <1 mm.

Der Isolator 30 kann aus Hartkunststoff oder Keramik bestehen. Damit ist gewährleistet, dass die Spitze 60 als Kathode/Anode fungiert und der Schaft 20 entsprechend die entgegengesetzte Polung aufweist. Der Isolator 30 weist dabei vorzugsweise eine Dicke von 5mm bis 10mm auf.

### Elektrische Leitungen 40 und 50

Dabei umfasst der Schaft 20 eine in den Schaft integrierte erste elektrische Leitung 40 mit einer ersten Elektrode 410. Dabei ist die erste Elektrode 410 an der Außenseite 260 des Schaftes 20 an dessen zweiten Ende 240 vor dem Isolator 30 angeordnet. Diese erste elektrische Leitung 40 ist dabei mit einem ersten Pol 1210 des Adapters 120 des Anschlusses 10 so verbindbar, dass Strom über die Reinigungsvorrichtung 1, dem ersten Pol 1210 und der ersten Elektrode 410 fließen kann.

In einer besonderen Ausführungsform wird der Schaft 20 selbst als erste Elektrode 410 angelegt. Hier kann zum Schutz eine nichtleitende Isolierschicht aufgebracht werden, so dass nur am vorderen Ende, d.h. vor dem Isolator eine leitfähige Zone vorhanden ist.

Weiterhin umfasst die Reinigungsvorrichtung 1 eine zweite elektrische Leitung 50 mit einer zweiten Elektrode 510. Dabei ist die zweite Elektrode 510 hinter dem elektrischen Isolator 30 angeordnet. Dabei ist die zweite elektrische Leitung 50 so angeordnet, dass sie den Isolator 30 entlang der Bohrung 310 durchquert.

Die zweite elektrische Leitung 50 ist mit einem zweiten Pol 1220 des Adapters 120 des Anschlusses 10 so verbindbar, dass Strom von der ersten elektrischen Leitung 40 über die erste Elektrode 410, das Fluid und die zweite Elektrode 510 zur zweiten elektrischen Leitung 50 fließen kann. Die Stromrichtung kann dabei entgegengesetzt sein.

Die Elektrode 510 ist vorzugsweise unmittelbar (Abstand 0mm) hinter dem Isolator 30 angeordnet.

Die Elektrode 510 ist unabhängig von ihrem Abstand zur Isolationsschicht vorzugsweise als Ringelektrode ausgebildet. Dabei ist ihr Durchmesser vorzugsweise geringer als der Durchmesser des Isolators 30 bzw. der Spitze 60, um einen Schutz vor Missempfindungen zu gewährleisten.

Die elektrischen Leitungen 40, 50 sind dabei beispielsweise in Form von isolierten Kupfer- und/oder Aluminiumdrähten, -kabeln oder -litzen ausgeführt. Die elektrischen Leitungen 40, 50 können innerhalb des Schaftes verlaufen oder in einer Rinne an dessen Rand eingegossen sein.

### Spitze 60

Weiterhin umfasst die Reinigungsvorrichtung 1 eine Spitze 60. Diese ist am Isolator befestigbar angeordnet. Weiterhin ist sie so mit der zweiten elektrischen Leitung 50 verbindbar ausgebildet, dass die Oberfläche der Spitze 60 als Elektrode 510 der zweiten elektrischen Leitung 50 dienen kann. Dazu ist wenigstens die Oberfläche der Spitze elektrisch leitfähig zumindest abschnittsweise ausgebildet.

Dabei besitzt die Spitze 60 ein erstes Ende 610, welches in Richtung Schaft 20 ausgerichtet ist und ein zweites Ende 620 welches sich auf der entgegengesetzten Seite der Spitze 60 befindet.

Die Spitze 60 weist an ihrem dem Schaft abgewandten Ende 620 vorzugsweise eine Öffnung zum Austritt von Fluid auf. Der Durchmesser dieser Öffnung beträgt üblicherweise ca. 0,1 - 0,5 mm.

Die Befestigung kann beispielsweise über Verschrauben erfolgen. Dabei weist beispielsweise der Isolator 30 ein Gewinde 320 auf, in welches die Spitze 60 geschraubt z.B. über einen Konus an ihrem ersten Ende 610 eingeführt werden kann. Alternative Befestigungen wie z.B. Presssitz oder Verkleben sind auch möglich. Durch die Befestigung zwischen dem Schaft 20 und dem Isolator 30 bzw. dem Isolator 30 und der Spitze 60 ist die gesamte Reinigungsvorrichtung 1 vorzugsweise mechanisch so stabil, dass die Spitze 60 durch den Schwingungsgenerator im Ultraschallbereich schwingen kann.

Die Spitze 60 ist an ihrem Ende 620 vorzugsweise abgerundet ausgebildet.

Dabei besteht die Spitze 60 vorzugsweise aus einem Edelstahl.

Alternativ kann sie auch aus einem anderen Metall bspw. aus elektrisch leitfähigem Titanoxid bestehen. Hochleistungskeramiken bzw. Polymere können auch verwendet werden, vorausgesetzt, dass sie elektrisch leitfähig sind. Alternativ weist die Spitze 60 eine elektrisch leitfähige Beschichtung auf, sodass die Spitze 60 an ihrer Oberfläche unabhängig von ihrem Kernmaterial als Elektrode 510 dienen kann. Ein geeignetes Material dafür ist Zirkonoxid.

Dieses Material ist gegen Säuren und Alkalilaugen sehr beständig und hat eine hohe Widerstandsfähigkeit gegen chemische, thermische und mechanische Einflüsse.

In einer besonderen Ausführungsform ist die Oberfläche der Spitze 60 auch aufgeraut bzw. diamantiert. Diese Aufrauhung umfasst vorzugsweise einen Bereich von 4-6 mm an ihrem Ende 620.

In einer weiteren Ausführungsform befindet sich wenigstens eine Öffnung 220 des Schafts 20 zwischen dem Schaft 20 und dem ersten Ende 610 der Spitze 60. Dabei ist die Spitze 60 so ausgebildet ist, dass Fluid von ihrem ersten Ende 610 zu ihrem Ende 620 fließen kann und die Spitze an ihrem zweiten Ende 620 verlassen kann.

Dabei weist die Spitze 60 vorzugsweise zusätzlich wenigstens eine Perforation 630 auf, so dass die vorhandene Oberfläche für den Austritt von Fluid vergrößert wird.

Der Schaft 20 und die Spitze 60 sind entweder einstückig oder zweistückig ausgebildet.

Vorzugsweise umfasst die Vorrichtung noch eine Olive 640. Die Olive 640 ist am dem Schaft 20 abgewandten hinteren Bereich der Spitze 60 angeordnet und dient dazu, dass ein Abstand zum Gewebe und zur Wurzeloberfläche im Dentalbereich 2 entsteht, der die Flüssigkeitsapplikation erleichtert. Die Spitze 60 kann in einer oder 2 Raumrichtungen gekrümmt sein um sie optimal an die sphärisch gekrümmten Wurzelflächen der Zähne adaptieren zu können.

### Verbindungselement 80

Weiterhin weist die Reinigungsvorrichtung 1 vorzugsweise ein Verbindungselement 80 auf, mit dem der Schaft 20 am Anschluss 10 befestigt werden kann. Dieses Verbindungselement 80 kann bspw. als ein Gewinde oder Bajonettverschluss ausgebildet sein. Weiterhin kann es noch ein Handstück 850 umfassen. Das Verbindungselement 80 umfasst eine elektrische Verbindung zum Adapter 120 des Anschluss 10. Vorzugsweise sind die Kontakte des Verbindungselements 260 am Anschluss mit Federdruck gelagert ausgebildet, so dass sie in Vertiefungen am Arbeitsende einrasten. Das Verbindungselement 80 weist eine Fluiddurchführung zwischen den Adapter 110 und der ersten Öffnung 210 des Schafts 20 auf. Die Verbindung ist vorzugsweise mechanisch so stabil, dass sie Ultraschallschwingungen übertragen kann.

Weiterhin umfasst das Verbindungselement 80 vorzugsweise einen Schalter 810 zum An- bzw. Abschaltung der Stromzuführung der Reinigungsvorrichtung 1. Eine bespielhafte Ausführungsform mit Verbindungselement 80 zeigt die Abbildung Fig.5.

### Ultraschallelement 70

In einer weiteren Ausführungsform umfasst die Reinigungsvorrichtung 1 noch ein Ultraschallelement 70.

Dazu weist das Ultraschallelement 70 wenigstens ein Antriebselement 710 aus einem piezoelektrischen oder magnetostriktiven Material auf, das bei Anlegen einer elektrischen Wechselspannung bzw. eines oszillierenden Magnetfelds eine lineare Oszillationsbewegung erzeugt. Die Antriebseinheit dient zur Bereitstellung von hoch frequenten Oszillationsbewegung in einem Frequenzbereich von 20 kHz bis zu 50 kHz. Weiterhin umfasst das Ultraschallelement 70 ein Steuerelement 720 über welches das Antriebselement 710 aktiviert bzw. deaktiviert werden kann.

Dieses Steuerelement 720 kann beispielsweise als ein Handschalter oder ein Fußschalter 90 ausgebildet sein. Das Ultraschallelement ist dabei so angeordnet, dass es die Oszillationen des Antriebselements 710 auf die Spitze 60 übertragen kann.

Das Ultraschallelement 70 ist vorzugsweise im Handstück 850 des Verbindungselements 80 angeordnet. Der Schaft 20 mit der Spitze 60 ist dabei so am Verbindungselements 80 aufgeschraubt oder anderweitig befestigt, sodass die Schwingungen aus dem Handstück 850 auf die Spitze 60 übertragen werden können. Eine bespielhafte Ausführungsform mit Ultraschallelement zeigt die Abbildung Fig.5.

### Weitere Ausführungsformen:

In einer weiteren Ausführungsform der Reinigungsvorrichtung 1 ist die Oberfläche des Schafts 20 als erster elektrischer Leiter 40 ausgebildet. Weiterhin ist die Oberfläche des Schafts 20 zumindest abschnittsweise freiliegend ausgebildet, sodass die freiliegenden Abschnitte der Oberfläche des Schafts 20 als Elektrode 410 dienen können. Vorzugsweise ist der Isolator 30 dabei so ausgebildet, dass er das zweite Ende 240 des Schafts 20 und das erste Ende 610 der Spitze 60 überdeckt. Dabei kann der Isolator 30 im Überdeckungsbereich, in welchem er den Schaft 20 überdeckt, wenigstens eine Peroration 340 aufweisen. So kann die Oberfläche des Schafts 20 unterhalb der wenigstens einen Perforation 340 als Elektrode 410 dienen.

Alternativ oder zusätzlich kann der Isolator im Überdeckungsbereich, in welchem er die Spitze 60 überdeckt, wenigstens seine Perforation 350 aufweisen. Der Überdeckungsbereich des Isolators 30 mit der Spitze 60 bildet damit einen Schirm, welcher die die Elektrode 510 vor einer direkten Berührung schützt.

Die wenigstens eine Perforation 350 kann dabei als eine ringförmige Kerbe ausgebildet sein, die zur Arbeitsspitze eine Öffnung aufweist und gleichzeitig als Austrittsstelle für Fluid fungiert.

### Ein Herstellungsverfahren für die Reinigungsvorrichtung 1:

Der Isolator 30 wird zunächst aus einer Zirkonronde gefräst und anschließend gesintert. Dann werden der Schaft 20 und die Spitze 60 und der Isolator 30 mit einem temperaturstabilen, sterilisierbaren Kleber (z. Bsp. Multitlink Hybrid) verbunden.

### Verfahrensbeschreibung:

Zum Betrieb der Reinigungsvorrichtung 1 wird diese in einem Schritt A zunächst über die Adapter 110 und 120 mit einer Fluid- bzw. einer Stromquelle verbunden.

Anschließend wird in einen Schritt B die Reinigungsvorrichtung 1 so positioniert, dass Strom von der zweiten elektrischen Leitung 50 mit der als Kathode betriebenen zweiten Elektrode 510 über das Fluid zur als Anode betriebenen ersten Elektrode 410 zur elektrischen Leitung 40 fließen kann.

Anschließend wird in einem Schritt C ein Fluid mit einer ausreichenden Konzentration beweglicher Ladungsträger über den Adapter 110 durch den Schaft 20 und dessen zweite Öffnung 220 in den zu reinigenden Dentalbereich 2 eingeleitet.

Grundbestandteil des Fluids ist beispielsweise ein Metallsalz in wässriger Lösung. Das Metallsalz liefert die Ionen für den Stromtransport.

Alternativ möglich ist auch die Verwendung einer organischen schwachem Säure z. B. Milchsäure. Andererseits ist auch die Verwendung von Laugen (z. B. aus Casein-Lösung) möglich. Auch Laugen sind biologisch wirksam, indem sie antimikrobiell wirken.

Durch die gezielte Wahl einer ausreichend hohen elektrischen Leitfähigkeit soll sichergestellt werden, dass der Stromfluss durch das Fluid und damit durch die behandlungsbedürftigen Teile und Komponenten im Dentalbereich 2, nicht aber durch das Körpergewebe des Patienten erfolgt, so dass eine Gefährdung des Patienten durch einen ungewollten Stromfluss durch Weichgewebe, Knochen, Blut und/oder andere Körpermaterialien minimiert werden kann. Die elektrische Leitfähigkeit des Fluids sollte dabei möglichst ein Vielfaches der elektrischen Leitfähigkeit von Blut, Knochen, Weichgewebe, Fettgewebe oder anderen Körpermaterialien aufweisen. Daher sollte die elektrische Leitfähigkeit des Fluids einen Wert von mindestens 30 mS/cm aufweisen.

Das Fluid besteht in einer beispielhaften Zusammensetzung aus einer Hydroxycarboxylsäure (Laktat, Glykolsäure, Äpfelsäure, Zitrat) in gepufferter Salzlösung. Alternativ kann eine leichte Lauge verwendet werden (0,05 m NaOH in physiologischer NaCl-Lsg.).

Das Einleiten des Fluids geschieht dabei vorzugsweise kontinuierlich. Die Durchflussmenge kann dabei variabel an der Fluidquelle oder am Anschluss 10 eingestellt werden, um einen kontinuierlichen Fluss zu gewährleisten. Die Flussrate hängt dabei von der Größe des zu reinigenden Implantats und dem entsprechenden Adapter ab.

Anschließend wird zwischen dem ersten Pol 1210 und dem zweiten Pol 1220 am Adapter 120 eine Spannungsdifferenz angelegt. Die Spannung liegt dabei vorzugsweise zwischen 10 V und 20 V.

Dann fließen Ladungsträger von der als Minuspol dienenden zweiten Elektrode 1220 des Adapters 120 über die zweite Leitung 50, die als Kathode dienende zweite Elektrode 510 und das Fluid über die erste elektrische Leitung 40 zur als Pluspol dienenden ersten Elektrode 1210 des Adapters 120.

Dabei werden im Fluid Ionen erzeugt. Diese Ionen reagieren chemisch oder elektrochemisch mit dem Biofilm. Durch diese Reaktionen werden neue Stoffverbindungen geschaffen und/oder die Ionen selbst und/oder Teile dieser Ionen in den atomaren Zustand überführt. Dies führt zu einer Entfernung des Biofilms. Dies erfolgt solange bis der Biofilm hinreichend entfernt wurde.

Durch den Stromfluss wird Wasser in seine Komponenten Wasserstoff und Sauerstoff gespalten, d.h. an der Kathode bilden sich kleine Wasserstoffbläschen, die auch zu einer mechanischen Disruption des Biofilms führen.

Kommen zusätzlich Ultraschallschwingungen dazu, dann implodieren die kleinen Gasbläschen. Dieser sog. "Kavitationseffekt" hat ebenfalls eine starke Reinigungswirkung durch Disruption des Biofilms. Beim Betrieb der Reinigungsvorrichtung ist es möglich, die Richtung des Stromflusses umzukehren, d.h., dass aus der zweiten Elektrode 510 an der Spitze 60 eine Anode wird, dadurch bilden sich dort dann Gasbläschen, die aus O₂ bestehen und den Sauerstoffpartialdruck in der Tasche erhöhen. Dies hilft bei der Beseitigung der dort v.a. vorhandenen anaeroben Bakterien. Das Gerät kann rein zur elektrolytischen Reinigung eingesetzt werden, d.h. der Ultraschall ist abgeschaltet (z.B. über einen Fußschalter). Umgekehrt kann es als alleiniges Ultraschall-Gerät eingesetzt werden.

Im Gegensatz zur Anwendung an Metallimplantaten bilden sich in dem hier vorgestellten Ansatz die Gasbläschen nicht auf der zu behandelnden Oberfläche, sondern auf der Spitze 60 der Reinigungsvorrichtung 1.

Die elektrolytische Funktion lässt sich am Anschluss 10 beispielsweise über einen Druckschalter an- und abschalten.

Wird der Strom abgeschaltet, ist es vorteilhaft das Gerät auf konventionelle Kühlung (durch das Wasser/NaCl-Lösung) aus der Reinigungsvorrichtung 1 umzuschalten.

Die Stromstärke liegt zwischen 0,1 und 1A. Die elektrische Leistung zur Wasserspaltung/Gasbildung für die elektrolytische Reinigung wird vorzugsweise auf maximal 600mA bei 15V Gleichstrom begrenzt.

Die Ultraschallfunktion beispielsweise wird per Fußschalter ein- bzw. ausgeschaltet. Im Unterschied zu allen herkömmlichen Verfahren ergänzt und potenziert die Elektrolyse die lokale Reinigungswirkung und gleichzeitig die bakterizide Wirkung, sodass durch eine vollständige Elimination des bakteriellen Biofilms optimale Voraussetzungen für die Ausheilung von Erkrankung geschaffen werden.

### Ausführungsbeispiele

In einer bespielhaften **Ausführungsvariante** 1 handelt sich bei der Reinigungsvorrichtung um eine Spülkanüle, die an ihrer Spitze als Kathode wirkt, wobei an der Spitze die das Fluid (eine Elektrolyt- Lösung) austritt. Die Lösung wird mit einer Schlauch-Quetschpumpe (Peristaltic-Pumpe) aus einem Vorratsbehältnis als Fluidquelle in die Reinigungsvorrichtung 1 gepumpt. Diese Variante ermöglicht keine Ultraschallzuschaltung.

Durch die Strom- und Fluidzufuhr bilden sich am Ende 620 der Spitze feine Gasbläschen, die im Fluid aufperlen und zu einer mechanischen Zerstörung des Biofilms führen. Die Reinigungsvorrichtung kann vom Schlauchsystem getrennt und nach Thermodesinfektion wiederverwendet werden. Schlauchsystem und Vorratsbehälter sind als Einmalartikel ausgebildet und werden nicht wiederverwendet.

Der Hand- 810 bzw. Fußschalter 90 gibt den Stromfluss frei und aktiviert gleichzeitig die Peristaltic-Pumpe, d.h. eine Freigabe des Stromflusses ohne Fluidzufuhr ist ausgeschlossen.

In einer bespielhaften **Ausführungsvariante** 2 handelt sich bei der Reinigungsvorrichtung um ein Handstück mit integriertem Ultraschallelement 70, welches nach dem Einschalten mit Hilfe des Schalters 710 Ultraschallschwingungen erzeugt, sodass über den Schaft das Arbeitsende ebenfalls in Ultraschallschwingungen versetzt wird. Gleichzeitig wird über eine Pumpe (z. B. Peristaltic-Pumpe) Fluid an die Spitze des Arbeitsendes befördert. Ein zweiter Schalter 810 ermöglicht die Stromzufuhr, so dass die Arbeitsspitze als Kathode/Anode wirkt und elektrolytisch kleine Gasbläschen entstehen, die eine zusätzliche disruptive Wirkung entfalten. Mit Hilfe des Ultraschalls implodieren die Mikrobläschen. Dieser Kavitationseffekt ist ebenfalls entscheidend für die Reinigungswirkung.

### Abbildungslegenden und Bezugszeichenliste

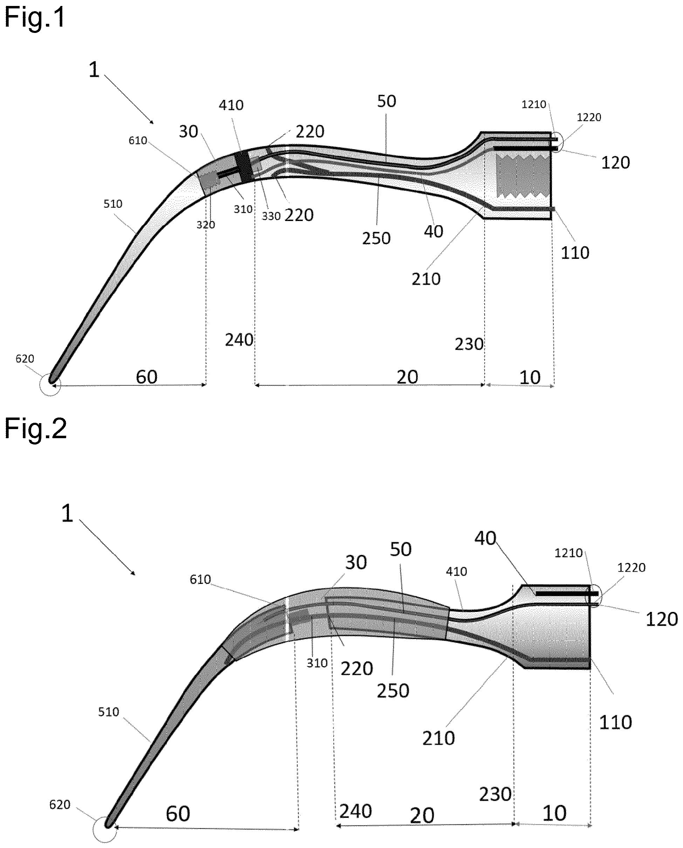

Fig. 1 zeigt schematisch den Aufbau der Reinigungsvorrichtung 1 mit gekrümmter Spitze 60.
Fig.2 zeigt schematisch den Aufbau einer Ausführungsform der Reinigungsvorrichtung 1 bei welcher die Oberfläche des Schafts 20 als erster elektrischer Leitung 40 und als Elektrode 410 ausgebildet ist. Hierbei ist der Isolator so ausgebildet, dass er das zweite Ende 240 des Schafts 20 und das erste Ende 610 der Spitze 60 überdeckt.

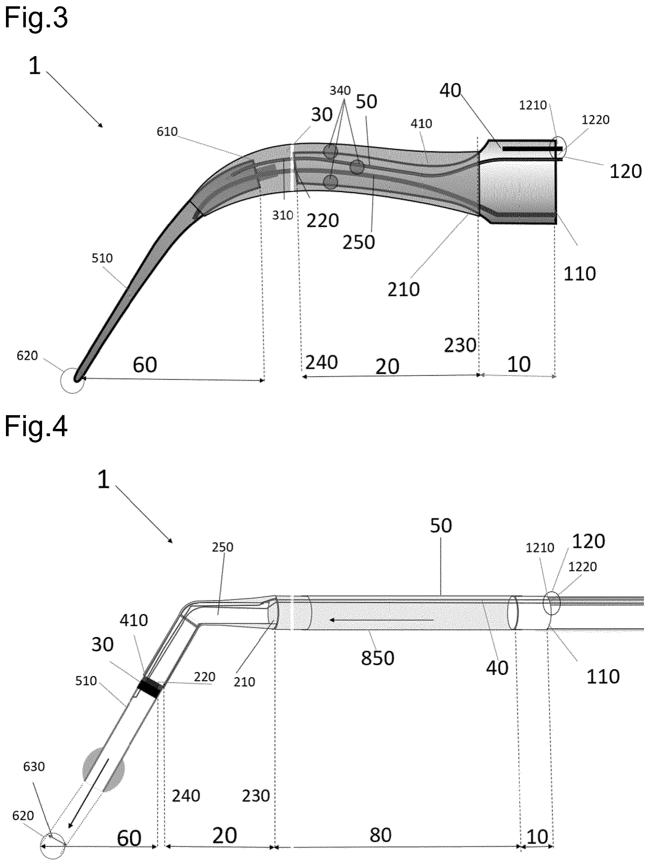

Fig.3 zeigt schematisch den Aufbau einer Ausführungsform der Reinigungsvorrichtung 1, bei welcher die Oberfläche des Schafts 20 als erster elektrischer Leitung 40 und als Elektrode 410 ausgebildet ist. Hierbei ist der Isolator so ausgebildet, dass er das zweite Ende 240 des Schafts 20 und das erste Ende 610 der Spitze 60 überdeckt. Hierbei weist der Isolator im Überdeckungsbereich zum Schaft 20 mehrere Perforationen 340 auf.
Fig. 4 zeigt schematisch den Aufbau der Reinigungsvorrichtung 1 mit gerader Spitze 60 und einem Verbindungselement 80 und einem Handstück 850. Die Pfeile zeigen die Flussrichtung des Reinigungsfluides im Betrieb.
Fig.5 zeigt schematisch den Aufbau der Reinigungsvorrichtung 1 mit gerader Spitze 60 und einem Verbindungselement 80 und einem Handstück 850. Diese Ausführungsform weist zusätzlich ein Ultraschallelement 70 mit einem Antriebselement 710, ein Steuerelement 720 und einen Schalter 730 zum An- bzw. Abschalten des Ultraschallelement 70 auf. Die Pfeile zeigen die Flussrichtung des Reinigungsfluides im Betrieb.
Fig. 6 zeigt die Reinigungsvorrichtung 1 im betriebsbereiten Zustand mit einem Fußschalter 90.

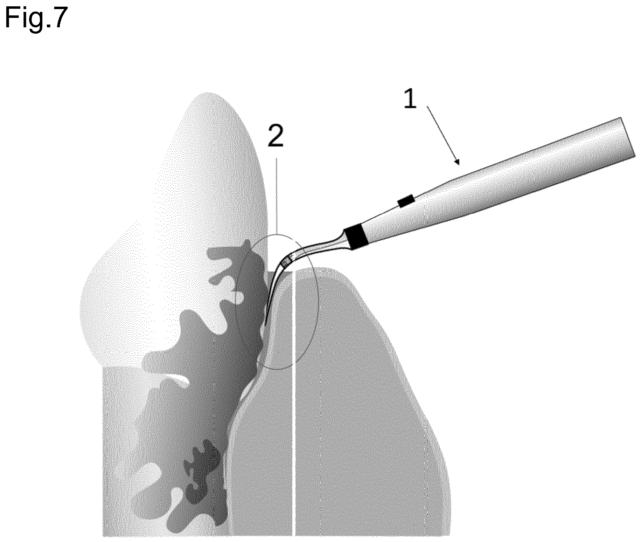

Fig. 7 zeigt beispielhaft die Verwendung der Reinigungsvorrichtung 1 zur Entfernung von Biofilmen in einem Dentalbereich 2.

### Bezugszeichen

1 Reinigungsvorrichtung
10 Anschluss
110 erster Adapter
120 zweiter Adapter
1210 erster elektrischer Pol am zweiten Adapter
1220 zweiter elektrischer Pol am zweiten Adapter
20 Schaft
210 erste Öffnung des Schafts
220 zweite Öffnung des Schafts
230 erstes Ende des Schafts
240 zweites Ende des Schafts
250 Hohlraum des Schafts
30 Isolator
310 Bohrung durch den Isolator
320 Gewinde des Isolators
330 Konus des Isolators
340 Perforation im Überdeckungsbereich zum Schaft
350 Perforation im Überdeckungsbereich zur Spitze
40 erste elektrische Leitung
410 Elektrode der ersten elektrischen Leitung
50 zweite elektrische Leitung
510 Elektrode der zweiten elektrischen Leitung
60 Spitze
610 erstes Ende der Spitze
620 zweites Ende der Spitze
640 Olive
70 Ultraschallelement
710 Antriebselement
720 Steuerelement
80 Verbindungselement
810 Schalter am Verbindungselement
850 Handstück
90 Fußschalter
2 Dentalbereich

## Patentansprüche

1. Reinigungsvorrichtung (1) zur Entfernung von Biofilmen im Dentalbereich wenigstens umfassend
• einen Anschluss (10) geeignet zum Anschließen an eine Fluidquelle und an eine Stromquelle zur Bereitstellung von Fluid und elektrischer Energie
o wobei der Anschluss (10) einen ersten Adapter (110) zum Einbringen von Fluid aufweist, um die Reinigungsvorrichtung (1) mit Fluid zu versorgen und einen zweiten Adapter (120) zum Einbringen von elektrischer Energie in die Reinigungsvorrichtung (1) aufweist
• einen fluiddurchströmbaren Schaft (20) mit wenigstens einer ersten Öffnung (210) zum Verbinden des Schafts (20) mit dem Adapter (110) des Anschlusses (10) und wenigstens einer zweiten Öffnung (220) in Richtung eines zu reinigenden Dentalbereichs (2),
o wobei der Schaft (20) ein in Richtung des Adapters (10) ausgerichtetes erstes Ende (230) und ein dem ersten Ende entgegengesetztes zweites Ende (240) aufweist,
o wobei sich zwischen einer ersten Öffnung (210) und der wenigstens einen zweiten Öffnung (220) ein Hohlraum (250) befindet, durch welchen ein Fluid frei strömen kann, wobei der fluiddurchströmbare Schaft (20) dabei mit dem Anschluss (10) so verbindbar ist, dass ein Fluid durch den Adapter (110) über die erste Öffnung (210) und den Hohlraum (250) durch die wenigstens eine zweite Öffnung (220) strömen kann sodass ein fluidumspülter Bereich im Dentalbereich (2) entstehen kann,
• einen elektrischen Isolator (30), welcher am zweiten Ende (240) des Schaftes verbindbar angeordnet ist und eine Bohrung (310) aufweist, durch welche eine elektrische Leitung (50) und Fluid durch den Isolator (30) geführt werden kann,
• eine innerhalb des Schafts (20) angeordnete erste elektrische Leitung (40) mit einer ersten Elektrode (410), wobei die erste Elektrode (410) an der Außenseite (260) des Schafts (20) an dem zweiten Ende (240) des Schafts (20) vor dem elektrischen Isolator (30) angeordnet ist, wobei die erste elektrische Leitung (40) dabei mit einem ersten Pol (1210) des Adapters (120) des Anschlusses (10) so verbindbar ist, dass Strom über die Reinigungsvorrichtung (1) zwischen einer Stromquelle und der ersten Elektrode (410) fließen kann,
• eine innerhalb des Schafts (20) angeordnete zweite elektrische Leitung (50) mit einer zweiten Elektrode (510), wobei die zweite elektrische Leitung (50) mit einem zweiten Pol (1220) des Adapters (120) des Anschlusses (10) so verbindbar ist, so dass Strom von der ersten elektrischen Leitung (40) über die erste Elektrode (410), das Fluid und die zweite Elektrode (510) zur zweiten elektrischen Leitung (50) fließen kann
**wobei**
• die Reinigungsvorrichtung (1) weiterhin eine Spitze (60) aufweist, wobei die Spitze (60) am Isolator (30) befestigbar angeordnet ist, wobei die Spitze (60) so mit der zweiten elektrischen Leitung (50) verbindbar ausgebildet ist, dass sie als zweite Elektrode (510) dienen kann,
• die zweite elektrische Leitung (50) so angeordnet ist, dass sie den Isolator (30) entlang der Bohrung (310) durchquert und die zweite Elektrode (510) hinter dem elektrischen Isolator (30) angeordnet ist, sodass bei Betrieb der Reinigungsvorrichtung (1) im fluidumspülten Bereich im Dentalbereich (2) zwischen der ersten Elektrode (410) und der zweiten Elektrode (510) Strom fließen kann, wobei sich im Fluid reaktive Verbindungen und/oder Gase bilden können, welche Biofilme im zu reinigenden Dentalbereich (2) entfernen.

2. Reinigungsvorrichtung (1) gemäß Anspruch **1 dadurch gekennzeichnet, dass** diese weiterhin noch ein Verbindungselement (80) zwischen dem Anschluss (10) und dem Schaft (20) aufweist, welches so ausgebildet ist, sodass der Schaft (20) über das Verbindungselement (80) am Anschluss (10) befestigt werden kann.

3. Reinigungsvorrichtung (1) gemäß Anspruch 1 oder 2 **dadurch gekennzeichnet, dass** diese weiterhin noch ein Ultraschallelement (70) umfasst, wobei das Ultraschallelement (70) wenigstens ein Antriebselement (710) und ein Steuerelement (720) aufweist, wobei das Antriebselement (710) bei Anlegen einer elektrischen Wechselspannung bzw. eines oszillierenden Magnetfelds eine lineare Oszillationsbewegung erzeugt und über das Steuerelement (720) das Antriebselement (710) aktiviert bzw. deaktiviert werden kann, wobei das Ultraschallelement (70) dabei so angeordnet ist, dass es bei Betrieb der Reinungsvorrichtung (1) die Oszillationen des Antriebselement (710) auf die Spitze (60) übertragen kann, um so Ultraschallschwingen zu erzeugen.

4. Reinigungsvorrichtung (1) gemäß einem der Ansprüche 1 bis 3 **dadurch gekennzeichnet, dass** sich wenigstens eine Öffnung (220) zwischen dem Schaft (20) und dem ersten Ende (610) der Spitze (60) befindet und die Spitze (60) so ausgebildet ist, dass Fluid von ihrem ersten Ende 610 zu ihrem zweiten Ende (620) fließen und die Spitze (60) an ihrem zweiten Ende (620) verlassen kann.

5. Reinigungsvorrichtung (1) gemäß einem der Ansprüche 1 bis 4 **dadurch gekennzeichnet, dass** die Oberfläche des Schafts (20) als erste elektrische Leitung (40) ausgebildet ist, wobei die Oberfläche des Schafts (20) zumindest abschnittsweise freiliegend ausgebildet ist, sodass der wenigstens eine freiliegende Abschnitt der Oberfläche des Schafts (20) als Elektrode (410) dienen kann.

6. Reinigungsvorrichtung (1) gemäß Anspruch 5 **dadurch gekennzeichnet, dass** der Isolator (30) so ausgebildet ist, dass er das zweite Ende (240) des Schafts (20) und das erste Ende (610) der Spitze (60) überdeckt.

7. Reinigungsvorrichtung (1) gemäß Anspruch 6 **dadurch gekennzeichnet, dass** der Isolator (30) in wenigstens einem Bereich, in welchem er den Schaft (20) überdeckt wenigstens eine Perforation (340) aufweist, sodass die Oberfläche des Schafts unterhalb der wenigstens eine Perforation (340) als Elektrode (410) dienen kann.

8. Reinigungsvorrichtung (1) gemäß Anspruch 6 oder 7 **dadurch gekennzeichnet, dass** der Isolator (30) in wenigstens einem Bereich, in welchem er die Spitze (60) überdeckt wenigstens eine Perforation (350) aufweist, sodass die Oberfläche der Spitze (60) unterhalb der wenigstens einen Perforation (350) als Elektrode (510) dienen kann.

9. Reinigungsvorrichtung (1) gemäß einem der vorigen Ansprüche **dadurch gekennzeichnet, dass die** Spitze (60) eine elektrisch leitfähige Beschichtung aufweist, sodass die Spitze (60) an ihrer Oberfläche unabhängig von ihrem Kernmaterial als Elektrode (510) dienen kann.

## Claims

1. Cleaning device (1) for removing biofilms in the dental area, comprising at least
• a connection (10) suitable for connecting to a fluid source and to a power source for supplying fluid and electrical energy
o wherein the connection (10) has a first adapter (110) for introducing fluid to supply the cleaning device (1) with fluid and a second adapter (120) for introducing electrical energy into the cleaning device (1)
• a fluid-permeable shaft (20) with at least one first opening (210) for connecting the shaft (20) to the adapter (110) of the connection (10) and at least one second opening (220) in the direction of a dental area (3) to be cleaned,
o wherein the shaft (20) has a first end (230) oriented toward the adapter (10) and a second end (240) opposite the first end,
• wherein a cavity (250) is located between a first opening (210) and the at least one second opening (220), through which a fluid can flow freely, wherein the fluid-permeable shaft (20) can be connected to the connection (10) in such a way that a fluid can flow through the adapter (110) via the first opening (210) and the cavity (250) through the at least one second opening (220) so that a fluid-flushed area can be created in the dental area (2),
• an electrical insulator (30) which is connectably arranged at the second end 240 of the shaft and has a bore (310) through which an electrical conductor (50) and fluid can be guided through the insulator (30),
• a first electrical conductor (40) arranged inside the shaft (20) with a first electrode (410), wherein the first electrode (410) is arranged on the outside (260) of the shaft (20) at the second end (240) of the shaft (20) in front of the electrical insulator (30), wherein the first electrical conductor (40) can be connected to a first pole (1210) of the adapter (120) of the connection (10) so that current can flow via the cleaning device (1) between a power source and the first electrode (410),
• a second electrical conductor (50) arranged inside the shaft (20) with a second electrode (510), wherein the second electrical conductor (50) can be connected to a second pole (1220) of the adapter (120) of the connector (10) so that current can flow from the first electrical conductor (40) via the first electrode (410), the fluid, and the second electrode (510) to the second electrical conductor (50)
**wherein**
• the cleaning device (1) further comprises a tip (60), wherein the tip (60) is arranged to be attachable to the insulator (30), wherein the tip (60) is designed to be connectable to the second electrical conductor (50) so that it can serve as a second electrode (510),
• the second electrical conductor (50) is arranged so that it crosses the insulator (30) along the bore (310) and the second electrode (510) is arranged behind the electrical insulator (30) so that, when the cleaning device is in operation (1) in the fluid-flushed area in the dental area 2, current can flow between the first electrode (410) and the second electrode (510), whereby reactive compounds and/or gases can form in the fluid, which remove biofilms in the dental area (2) to be cleaned.

2. Cleaning device (1) according to claim 1, **characterized in that** it further comprises a connecting element (80) between the connection (10) and the shaft (20), which is designed so that the shaft (20) can be attached to the connection (10) via the connecting element (80).

3. Cleaning device (1) according to claim 1 or 2, **characterized in that** it further comprises an ultrasonic element (70), wherein the ultrasonic element (70) has at least one drive element (710) and one control element (720), wherein the drive element (710) generates a linear oscillatory movement when an alternating electrical voltage or an oscillating magnetic field is applied and can be activated or deactivated via the control element (720), wherein the ultrasonic element (70) is arranged such that, during operation of the cleaning device (1), it can transmit the oscillations of the drive element (710) to the tip (60) in order to generate ultrasonic vibrations.

4. Cleaning device (1) according to one of claims 1 to 3, **characterized in that** at least one opening (220) is located between the shaft (20) and the first end (610) of the tip (60), and the tip (60) is designed such that fluid can flow from its first end (610) to its second end (620) and leave the tip (60) at its second end (620).

5. Cleaning device (1) according to one of claims 1 to 4, **characterized in that** the surface of the shaft (20) is designed as a first electrical conductor (40), wherein the surface of the shaft (20) is designed to be exposed at least in sections so that the at least one exposed section of the surface of the shaft (20) can serve as an electrode (410).

6. Cleaning device (1) according to claim 5, **characterized in that** the insulator (30) is designed to cover the second end (240) of the shaft (20) and the first end (610) of the tip (60).

7. Cleaning device (1) according to claim 6, **characterized in that** the insulator (30) has at least one perforation (340) in at least one area in which it covers the shaft (20), so that the surface of the shaft below the at least one perforation (340) can serve as an electrode (410).

8. Cleaning device (1) according to claim 6 or 7, **characterized in that** the insulator (30) has at least one perforation (350) in at least one area in which it covers the tip (60), so that the surface of the tip (60) below the at least one perforation (350) can serve as an electrode (510).

9. Cleaning device (1) according to one of the preceding claims, **characterized in that** the tip (60) has an electrically conductive coating so that the tip (60) can serve as an electrode (510) on its surface regardless of its core material.

## Revendications

1. Dispositif de nettoyage (1) destiné à éliminer les biofilms dans le domaine dentaire, comprenant au moins
• un raccord (10) adapté pour être raccordé à une source de fluide et à une source d'énergie électrique afin de fournir du fluide et de l'énergie électrique
o le raccord (10) comportant un premier adaptateur (110) pour l'introduction de fluide afin d'alimenter le dispositif de nettoyage (1) en fluide et un deuxième adaptateur (120) pour l'introduction d'énergie électrique dans le dispositif de nettoyage (1)
• un corps (20) pouvant être traversé par le fluide, comportant au moins une première ouverture (210) pour relier le corps (20) à l'adaptateur (110) du raccord (10) et au moins une deuxième ouverture (220) en direction d'une zone dentaire (3) à nettoyer,
o la tige (20) présentant une première extrémité (230) orientée vers l'adaptateur (10) et une deuxième extrémité (240) opposée à la première extrémité,
o une cavité (250) étant située entre une première ouverture (210) et la au moins une deuxième ouverture (220), à travers laquelle un fluide peut s'écouler librement, la tige (20) traversable par un fluide pouvant être reliée au raccord (10) de telle sorte qu'un fluide puisse s'écouler à travers l'adaptateur (110) via la première ouverture (210) et la cavité (250) à travers au moins une deuxième ouverture (220), de sorte qu'une zone balayée par le fluide peut être créée dans la zone dentaire (2),
• un isolateur électrique (30) qui est disposé de manière à pouvoir être raccordé à la deuxième extrémité 240 de la tige et qui présente un alésage (310) à travers lequel un conducteur électrique (50) et un fluide peuvent être acheminés à travers l'isolateur (30),
• un premier conducteur électrique (40) disposé à l'intérieur de la tige (20) et comportant une première électrode (410), la première électrode (410) étant disposée sur la face extérieure (260) de la tige (20) au niveau de la deuxième extrémité (240) de la tige (20) avant l'isolateur électrique (30), le premier conducteur électrique (40) pouvant ainsi être connecté à un premier pôle (1210) de l'adaptateur (120) du raccord (10) de manière à ce que le courant puisse circuler entre une source de courant et la première électrode (410) via le dispositif de nettoyage (1),
• un deuxième conducteur électrique (50) disposé à l'intérieur de la tige (20) avec une deuxième électrode (510), le deuxième conducteur électrique (50) pouvant être connecté à un deuxième pôle (1220) de l'adaptateur (120) du raccord (10) de manière à ce que le courant puisse circuler depuis le premier conducteur électrique (40) via la première électrode (410), le fluide et la deuxième électrode (510) vers le deuxième conducteur électrique (50)
**selon lequel**
• le dispositif de nettoyage (1) comprend en outre une pointe (60), la pointe (60) étant disposée de manière à pouvoir être fixée à l'isolateur (30), la pointe (60) étant conçue pour pouvoir être reliée au deuxième conducteur électrique (50) de manière à pouvoir servir de deuxième électrode (510),
• le deuxième conducteur électrique (50) est disposé de manière à traverser l'isolateur (30) le long de l'alésage (310) et la deuxième électrode (510) est disposée derrière l'isolateur électrique (30), de sorte que, lors du fonctionnement du dispositif de nettoyage (1) dans la zone rincée par un fluide dans la zone dentaire 2, un courant peut circuler entre la première électrode (410) et la deuxième électrode (510), des composés et/ou des gaz réactifs pouvant se former dans le fluide, lesquels éliminent les biofilms dans la zone dentaire (2) à nettoyer.

2. Dispositif de nettoyage (1) selon la revendication 1, **caractérisé en ce qu'il** comprend en outre un élément de liaison (80) entre le raccord (10) et la tige (20), qui est conçu de telle sorte que la tige (20) peut être fixée au raccord (10) via l'élément de liaison (80).

3. Dispositif de nettoyage (1) selon la revendication 1 ou 2, **caractérisé en ce qu'il** comprend en outre un élément à ultrasons (70), l'élément à ultrasons (70) comportant au moins un élément d'entraînement (710) et un élément de commande (720), l'élément d'entraînement (710) générant un mouvement oscillatoire linéaire lorsqu'une tension électrique alternative ou un champ magnétique oscillant lui est appliqué et pouvant être activé ou désactivé par l'intermédiaire de l'élément de commande (720), l'élément ultrasonique (70) étant disposé de manière à pouvoir transmettre, lors du fonctionnement du dispositif de nettoyage (1), les oscillations de l'élément d'entraînement (710) à la pointe (60) afin de générer des vibrations ultrasoniques.

4. Dispositif de nettoyage (1) selon l'une des revendications 1 à 3, **caractérisé en ce qu'**au moins une ouverture (220) est située entre la tige (20) et la première extrémité (610) de la pointe (60) et **en ce que** la pointe (60) est conçue de telle sorte que le fluide s'écoule de sa première extrémité (610) vers sa deuxième extrémité (620) et puisse quitter la pointe (60) à sa deuxième extrémité (620).

5. Dispositif de nettoyage (1) selon l'une des revendications 1 à 4, **caractérisé en ce que** la surface de la tige (20) est conçue comme un premier conducteur électrique (40), la surface de la tige (20) étant conçue de manière à être exposée au moins par sections, de sorte que la au moins une section exposée de la surface de la tige (20) puisse servir d'électrode (410).

6. Dispositif de nettoyage (1) selon la revendication 5, **caractérisé en ce que** l'isolateur (30) est conçu de manière à recouvrir la deuxième extrémité (240) de la tige (20) et la première extrémité (610) de la pointe (60).

7. Dispositif de nettoyage (1) selon la revendication 6, **caractérisé en ce que** l'isolateur (30) présente au moins une perforation (340) dans au moins une zone dans laquelle il recouvre la tige (20), de sorte que la surface de la tige sous la au moins une perforation (340) peut servir d'électrode (410).

8. Dispositif de nettoyage (1) selon la revendication 6 ou 7, **caractérisé en ce que** l'isolateur (30) présente au moins une perforation (350) dans au moins une zone dans laquelle il recouvre la pointe (60), de sorte que la surface de la pointe (60) située sous la au moins une perforation (350) peut servir d'électrode (510).

9. Dispositif de nettoyage (1) selon l'une des revendications précédentes,
**caractérisé en ce que** la pointe (60) présente un revêtement électriquement conducteur, de sorte que la pointe (60) peut servir d'électrode (510) à sa surface, indépendamment de son matériau de base.
